# EUROPEAN PATENT APPLICATION

(11) **EP 3 838 113 A1**
(43) Date of publication of application: **23.06.2021**
(21) Application number: 19306731.1
(22) Date of filing: 20.12.2019
(51) Int. Cl.: A61B 3/00, A61B 3/06

(54) **A DEVICE FOR DETERMINING A CHANGE IN THE VISUAL COMFORT OF A USER**

(71) Applicant: ESSILOR INTERNATIONAL, 94220 Charenton-Le-Pont (FR)
(72) Inventor: DING, Shuang, 94220 CHARENTON-LE-PONT (FR); PERROT, Stéphane, 94220 CHARENTON-LE-PONT (FR)
(74) Representative: Ipsilon

(57) **Abstract**

The invention concerns a device and a method for determining a change in the visual comfort of a user, the device comprising:
- at least one light source for stimulating at least one eye of the user;
- a sensing unit facing at least one eye area (44) of the user when the device is worn by the user, the sensing unit being configured to remotely acquire at least one signal (46) representative of at least one characteristic of said at least one eye area; and
- a controller configured to determine a change in the visual comfort of the user depending on a variation of said at least one signal acquired by the sensing unit.

## Description

The invention relates to the field of visual comfort determination of a user. Particularly, the invention is directed to a device for determining a change in the visual comfort of a user. The invention further concerns a method for determining a change in the visual comfort of the user using such a device.

Comfort and visual acuity of a subject may vary depending on the light stimulation which is experienced by this subject. The alteration of the comfort and visual acuity with regard to a light stimulus is specific to each subject. Therefore, it is important to evaluate the visual comfort for each subject when providing him with the most appropriate lens or device that may take into account the visual comfort of the subject, as a light environment.

It is known to determine a visual discomfort of a subject or user using subjective methods. In these methods, the user is stimulated with a light source and indicates when a visual discomfort is felt. However, these methods are too dependent on the user's judgment and thus only makes it possible to partially determine the visual discomfort of the user.

It is also known to determine a visual discomfort of a user using objective methods. In these methods, electrodes are positioned on the head of the user to detect electroencephalogram signals or muscles activity, particularly in the eye areas. However, these methods are very invasive, expensive and complex to implement. Indeed, a physician is necessary to dispose the electrodes at the right place and analyze the results so that opticians and optometrists cannot use these methods.

A problem that the invention aims to solve is thus to provide a non-invasive and cost-effective device for determining a visual discomfort with an improved accuracy.

To solve this problem, the invention provides a device for determining a change in the visual comfort of a user, comprising:
- at least one light source for stimulating at least one eye of the user;
- a sensing unit facing at least one eye area of the user when the device is worn by the user, the sensing unit being configured to remotely acquire at least one signal representative of at least one characteristic of said at least one eye area; and
- a controller configured to determine a change in the visual comfort of the user depending on a variation of said at least one signal acquired by the sensing unit.

This device allows to determine a change in the visual comfort of a user depending on a variation of a signal representative of at least one characteristic of an eye area of the user remotely acquired. In doing so, the device thus allows to remotely and objectively evaluate a visual discomfort of the user when stimulated by a light source. A physician is therefore not required to implement the device because no electrode or measurement device disposed on the skin of the user is needed.

The characteristic is selected to be representative of a visual discomfort so that when a variation of the signal occurs it can be determined that a change in the visual comfort is experienced by the user. This change in the visual comfort can be thus matched with the light conditions provided at the time the change occurred. As an example, a light sensitivity threshold of the user may be determined when a characteristic of the eye area varies, as a position of the pupil or the eyelid. This variation can be thus associated to a response of the eye area to the light emitted toward the eye area representative of a visual discomfort.

According to an embodiment of said device, said sensing unit is a contactless sensing unit with regard to said at least one eye area for the acquisition of said at least one signal.

By providing a contactless sensing unit, it is therefore possible to perform a remote acquisition of said at least one signal. This signal acquisition is therefore non-invasive because the sensing unit is not in contact with the skin of the user for acquiring the signal.

According to an embodiment of said device, said sensing unit comprises a transmitter for transmitting a first signal toward said at least one eye area, and a receptor for receiving a second signal corresponding to the first signal reflected by said at least one eye area.

The acquisition of said at least one signal is therefore performed by receiving the response of a signal transmitted by the sensing unit toward the eye area. The comparison between the transmitted signal and the received signal allows the controller to determine a variation of said at least one signal.

According to an embodiment of said device, said controller is configured to determine a variation of intensity and/or frequency of the second signal and to correlate said variation to a response of the at least one eye area to a light stimulus provided by said at least light source.

Indeed, the controller allows to determine that a characteristic of the eye area reach a maximum value, as a threshold position value in the case of an eyelid closing the eye, or a short variation of the signal which can be also representative of a visual discomfort. Indeed, a slight movement of an eyelid or of the pupil can be representative of a visual discomfort, particularly a first level of visual discomfort.

According to an embodiment of said device, said characteristic of said at least one eye area comprises at least one among a position of at least one eyelid, a position of the pupil and a size of the pupil.

The sensing unit thus allows the controller to determine a change in the visual comfort depending on the closing/opening level of at least one eyelid and/or the response of the pupil. It allows to enhance the determination of a change in the visual comfort by making the device configured to detect different kind of response from the user. Indeed, the response to a light stimulus can be very different depending on the users.

According to an embodiment of said device, said controller is configured to perform a calibration of the sensing unit to obtain a first and a second values of the second signal respectively corresponding to a first and a second states of the user's eye area.

This calibration allows to set a reference value for a given state of the user's eye area. The controller is then able to compare a acquired value with the reference value so as to determine whether the user's eye area is in said given state. As an example, if the first and second states are a closing and an opening states of the eye, the controller can thus determine whether the eye of the user is closed or opened. A plurality of calibration values can be provided to calibrate intermediate position of the eyelids to make the determination more accurate and repeatable.

According to an embodiment of said device, the sensing unit further comprises a switch reachable by the user to provide during said calibration to the controller a signal representative of at least one characteristic of said at least one eye area when at least one user's eye area is in a first state and a signal representative of at least one characteristic of said at least one eye area from the switch when the user's area is in a second state .

According to an embodiment of said device, said sensing unit comprises at least one infrared sensor facing said at least one eye area when the device is worn by the user.

The infrared sensors are cheap and easy to implement to a support. Furthermore, the wearable configuration of the device enables easy handling of the device so that a determination method may be performed quickly. Easy and practical handling allows to consider new uses for the device. Indeed, said device may be used directly by the eye care professional without the need of a bulky measurement machine. Said device may also be used by the user himself at home or in various conditions, for example by measuring its light sensitivity threshold at different times of the day, months and/or years.

According to an embodiment of said device, said sensing unit comprises an image acquisition system for acquiring at least one image of said at least one eye area.

An image acquisition system allows to provide a signal with more information to process so that a more detailed determination may be made.

According to an embodiment of said device, said image acquisition system is configured to acquire at least one tridimensional image of said at least one eye area.

With the relevant post-processing, tridimensional image allows to refine the determination and thus make it even more accurate.

According to an embodiment of said device, the device is a binocular device, said at least one light source being configured to stimulate at least one eye of the user.

According to an embodiment of said device, said at least one eye area comprises at least one among lower and upper eyelids, an eyebrow, an eyelash and an eye.

The invention further proposes a method for determining a change in the visual comfort of the user, comprising the following steps:
- providing a device, comprising:
   - at least one light source for stimulating at least one eye of the user;
   - a sensing unit facing at least one eye area of the user when the device is worn by the user, the sensing unit being configured to remotely acquire at least one signal representative of at least one characteristic of said at least one eye area; and
   - a controller configured to determine a change in the visual comfort of the user depending on a variation of said at least one signal acquired by the sensing unit,
- positioning the device on the user's head such that said sensing unit faces at least one user's eye;
- emitting light toward at least one eye of the user;
- remotely acquiring at least one measuring signal representative of at least one characteristic of said at least one eye area;
- determining a change in the visual comfort of the user depending on a variation of said at least one measuring signal acquired.

According to an embodiment of said determining method, the sensing unit comprises a switch reachable by the user, said method further comprising a calibration sequence comprising the following steps prior to the remotely acquiring step:
- receiving a signal representative of at least one characteristic of said at least one eye area from the switch when at least one user's eye area is in a first state,
- remotely acquiring a first calibration signal representative of said first state of said at least one eye area,
- determining a first value corresponding to the first state of the user's eye depending on the first calibration signal,
- receiving a signal representative of at least one characteristic of said at least one eye area from the switch when the user's area is in a second state,
- remotely acquiring a second calibration signal representative of said second state of said at least one eye area,
- determining a second value corresponding to the second state of the user's eye depending on the second calibration signal,
- recording the first and second values.

According to an embodiment, said determining method is a computer-implemented method.

The invention is described in more detail below by way of the figures that show only one preferred embodiment of the invention.
Figure 1 schematically shows a perspective view of one side of a binocular optoelectronic device.
Figure 2 schematically shows a perspective view of another side of the binocular optoelectronic device.
Figure 3 schematically shows perspective view of the binocular optoelectronic device partly disassembled.
Figure 4 schematically shows a first infrared signal emitted from an infrared sensor toward the eye area of a user and a second infrared signal reflected by the eye area toward the infrared sensor, according to a first embodiment of a sensing unit.
Figures 5 and 6 schematically show an interface of an external terminal and graphs illustrating information acquired by the infrared sensor.
Figures 7 to 10 schematically show different steps of an image processing to determine an eye corner angle, according to a second embodiment of the sensing unit.
Figure 11 schematically shows a graph illustrating the evolution of an eye corner angle over time.
Figure 12 schematically shows a tridimensional image of the user's eye area according to a third embodiment of the sensing unit.

The present invention provides a device and a method for determining a change in the visual comfort of a user when stimulating with predetermined light conditions. Said device is preferably an optoelectronic device, i.e. an electronic device that source, detect and/or control light.

By "change in the visual comfort" of the user, what is meant is an alteration of the visual comfort experienced by the user, in the form of a visual discomfort or a modification of the visual performance.

The visual comfort can be associated to the light sensitivity of the user. The device may be thus configured to determine a light sensitivity threshold of the user by monitoring the response of the user's eye areas when subjected to a given light environment. By "sensitivity to light" of the user, what is meant is any relatively intense and prolonged reaction or modification of comfort or visual performance in relation to a temporary or continuous light flux or stimuli.

The quantity representative of the sensitivity of the eye of the user to said characteristic light flux is the light sensitivity threshold. It can be determined by measuring physical responses experienced by the user or any action of the user representative of its discomfort or visual perception. It allows the visual performance and/or visual discomfort experienced by the user to be determined objectively.

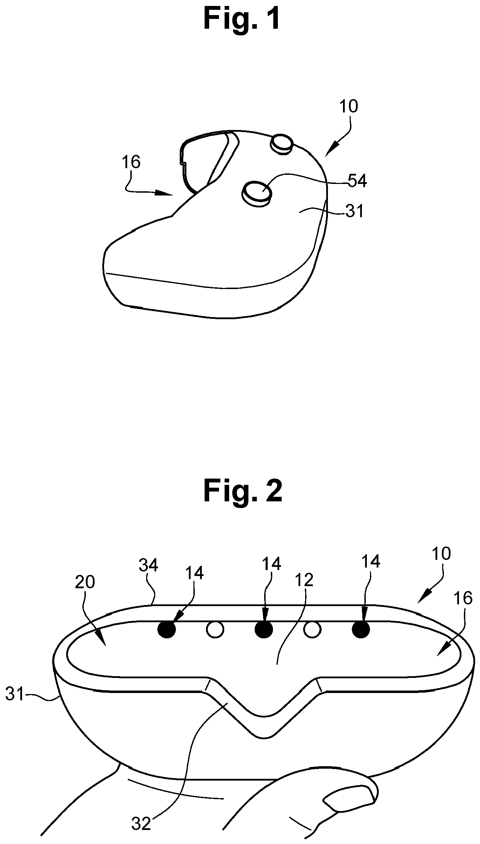

As shown on figure 1, a device 10 is configured to face an eye area of a user in use. Particularly, the device 10 is a binocular device so that it is configured to face each eye area of the user in use. Alternatively, the device 10 may be monocular and configured to face only one eye area of the user.

Said device 10 is preferably wearable by the user. In other words, dimensions and weight of the device 10 are configured to make it possible for a user to handle it in front of its eyes using supporting means. Said supporting means may be its hands so that the user handles the device 10 as binoculars. Alternatively, supporting means may be means for fastening the device 10 to the user's head as straps able to surround the user's head or spectacle arms positioned onto the user's ears. Alternatively, supporting means may be a support leg configured to sit on a table or on the ground. Furthermore, the device 10 comprises an accumulator 43 to be self-sufficient in energy.

As shown on figures 1 and 2, the device 10 comprises at least one light source 14 for stimulating at least one eye of the user. Said light source 14 is preferably lodged in a cavity 16 formed by a casing 31 of the device 10. Said at least one light source 14 may be combined with a diffuser 12 disposed within the cavity 16 in front of the user's eyes to provide a diffused light. In this case, the light source 14 emits light toward the diffuser 12. Alternatively or in combination, the light source 14 may be positioned to emit light directly toward one or both eyes of the user. Hence, the device 10 may be configured to expose the user to either a homogeneous or punctual light, or both simultaneously.

Light source 14 preferably comprises at least one light-emitting diode (LED) able to have variable light spectrum as RGB LEDs (Red-Green-Blue light emitting diodes) or RGB-W LEDs ((Red-Green-Blue-White light emitting diodes). Alternatively, said light source 14 may be configured to provide a predetermined single white light spectrum or, alternatively, a spectrum having all visible radiations with substantially the same intensity, in contrast with a spectrum having peaks. Said at least one light source 14 is preferably controlled with a constant current to obtain a constant light flux coming out said at least one light source 14. Providing the user with a constant light flux allows to reduce or avoid biological effects disturbances compared to light sources controlled with Pulse Width Modulation (PWM).

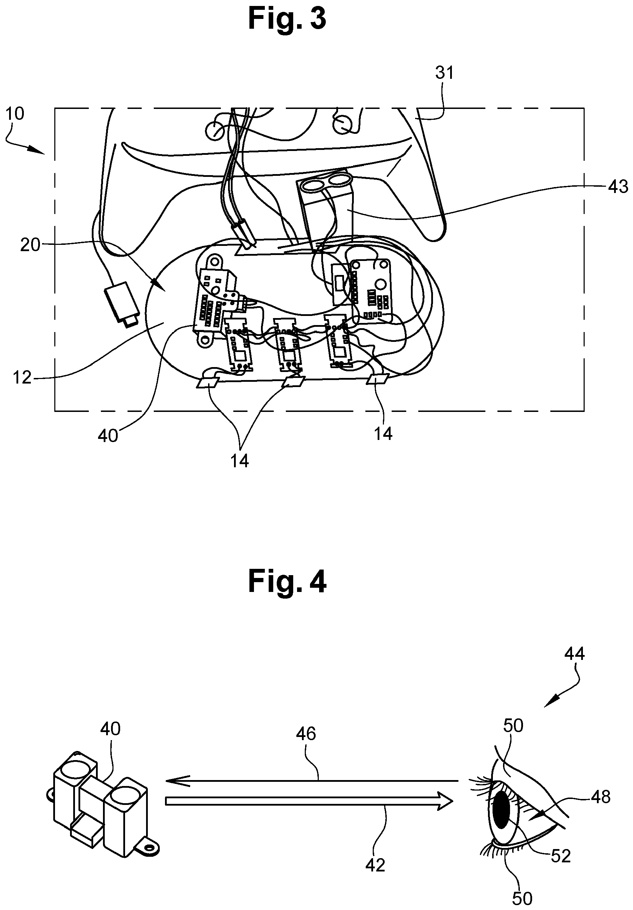

As shown on figure 3 which illustrates the device 10 in a disassembled manner, the device 10 further comprises a sensing unit 20 configured to face an eye area of the user when the device 10 is worn by the user. Alternatively, the sensing unit may be configured to face each eye area of the user, notably when the device 10 is binocular. The eye area comprises at least one among lower and upper eyelids, an eyebrow, an eyelash and an eye.

The sensing unit 20 is configured to remotely acquire at least one signal representative of at least one characteristic of said at least one eye area. By a "remote" acquisition, we meant that the signal is acquired without positioning an electrode or a measurement element onto the eye area or the skin of the user. In other words, the acquisition of the signal is contactless between the eye area and the sensing unit 20. Particularly, the acquisition of said at least one signal may be performed at a distance greater than or equal to 1 cm. In a preferred embodiment, only the casing 31 contacts the user for positioning and supporting the device 10 onto the user's head.

Said at least one characteristic of said at least one eye area comprises at least one among a position of at least one eyelid, a position of the pupil and a size of the pupil.

When the acquired signal concerns the position of at least one eyelid, the sensing unit 20 is thus able to acquire a signal representative of a closing/opening state of the eye. Furthermore, the position of one or two eyelids allows to determine a frequency of blink, an amplitude of blink, a duration of blink and different patterns of blink.

When the acquired signal concerns the position of the pupil, the sensing unit 20 is able to acquire a signal representative of the position of the eye itself. Then, when the acquired signal concerns the size of the pupil, the sensing unit 20 is able to acquire a signal representative of the dilatation/retractation level of the pupil.

A variation of one or more of the position of at least one eyelid, the position of the pupil and the size of the pupil can be representative of a visual discomfort. Therefore, a variation of one of these characteristics allows to determine a change in the visual comfort. It is then possible to correlate the light conditions experienced at the time the variation occurs to a change in the visual comfort. In doing so, a light sensitivity threshold of the user can be determined objectively.

The device 10 further comprises a controller 22 connected to the sensing unit 20 to receive the acquired signal from the sensing unit 20. The controller 22 is configured to determine a change in the visual comfort of the user depending on a variation of said at least one signal acquired by the sensing unit 20. The controller 22 is configured to process the signal acquired by the sensing unit 20 to determine a change in the visual comfort. This processing can comprise applying fast Fourier transform to transform discrete time domain data in the frequency domain. This determination is preferably performed following a light stimulus provided by said at least light source to correlate the change in the visual comfort to the light conditions experienced by the user.

The controller 22 may be fully or partly embedded within the casing 31. Indeed, the controller 22 may be partly disposed within an external terminal.

In use, the device 10 is positioned on the user's head so that the sensing unit 20 faces at least one eye area of the user. The device 10 may further comprise a cutout 32 configured to cooperate with the nose of the user to position the diffuser 12 in front of the user's eyes. To precisely position device 10 with regard to user's eye areas, the device 10 may also comprise a positioning surface 34 disposed at the opposite of the cutout 32 with respect to the cavity 16 to contact the user's forehead.

Light is emitted toward at least one eye of the user to provide him with predetermined light conditions to glare the user. A measurement sequence may be performed comprising three measurement steps. The acquisition of the signal by the sensing unit 20 is performed during the measurement steps.

The first measurement step is a continuous light emission to induce an illuminance from a minimum to a maximum values increasing the illuminance by stages, e.g. from 25 Lux to 10211 Lux. For example, the light emission may start with an illuminance of 25 Lux for 5 seconds to adapt the eye to the light condition and cancel all previous light exposure before the measurement and then continue with an increase of the illuminance of 20% each second to the maximum illuminance. In a more general way, the light may be emitted to induce an illuminance varying from 25 Lux to 15000Lux. This first measurement step is performed with warm light.

The second measurement step is performed identically to the first measurement step but with cold light.

Then, the third measurement step is a flashing light emission to induce an illuminance from a minimum value to a maximum value increasing the illuminance by stages, e.g. from 25 Lux to 8509 Lux. The illuminance of the flashing light emission is preferably increased by at least 30%, preferably by 40%, most preferably by at least 44%. Before and between each flash light emission, the user is subjected to a light emission lower than the minimum value of illuminance of the flashing light emission, e.g. 10 Lux. The time of each flashing light emission is preferably 0,5s and the time between each flashing light emission is preferably 2s.

During the measurement steps, at least one measuring signal representative of at least one characteristic of said at least one eye area 44 is remotely acquired by the sensing unit 20. The measuring signal is continuously or intermittently transmitted to controller 22. The controller 22 then process the measuring signal to determine a change in the visual comfort of the user depending on a variation of said at least one signal acquired.

The determining method may also comprise a calibration sequence to provide reference values specific to the user for which the visual comfort is determined. Prior the measurement steps wherein the user is stimulated with light, the user is asked to perform the calibration sequence by following instructions, as for example closing or opening his eyes. The sensing unit 20 preferably comprises a switch 54 positioned reachable by the user. The switch 54 is for example positioned on the upper portion of the casing 31.

The user is asked to put his eye area in a first state, for example a closing state of the eyelids, and then press the switch 54. A signal representative of at least one characteristic is received by the controller 22 from the switch 54 when the user's eye area is in the first state. This signal allows the controller 22 to know that the user's eye area is in the first state. The sensing unit 20 remotely acquires a first calibration signal representative of said first state of said at least one eye area. A first value corresponding to the first state of the user's eye is then determined depending on the first calibration signal.

Then, the user is asked to put his eye area in a second state, for example an opening state of the eyelids, and then press the switch 54. A signal representative of at least one characteristic is received by the controller 22 from the switch 54 when the user's eye area is in the second state. This signal allows the controller 22 to know that the user's eye area is in the second state. The sensing unit 20 remotely acquires a second calibration signal representative of said second state of said at least one eye area. A first value corresponding to the second state of the user's eye is then determined depending on the second calibration signal.

These first and second values may be an intensity of an infrared signal reflected by the eye area, an angle formed by the eyelids or a position of an eyelid.

The first and second values are then recorded in a memory connected to the controller 22. These first and second values can be then used as reference values in the measurement steps when determining a change in the visual comfort of the user. Indeed, a reference value representative of a predetermined state of the user's eye area, as a closing state of the eyelids, allows to accurately determine this change in the visual comfort by comparing the intensity of the signal acquired to the reference values. If the intensity of the signal acquired by the sensing unit 20 matches one of the reference values, contemplating a potential margin of error, the controller 22 can determine if the eye area is in the first or second state when the signal is acquired. Following the example above, a closing or opening state of the eye area can thus be determined by the controller 22.

Different embodiments of the sensing unit 20 that can be contemplated are described hereinafter. A first embodiment of the sensing unit 20 is directed to using infrared rays reflected by the eye area, a second embodiment is directed to taking and processing bidimensional images of the eye area and a third embodiment is directed to taking and processing tridimensional images of the eye area.

According to the first embodiment, the sensing unit 20 comprises an infrared sensor 40 facing one eye area of the user when the device 10 is worn by the user. In other words, the infrared sensor 40 is positioned on the casing 31 so as to face the eye area of the user. As shown on figure 3, the infrared sensor 40 may be disposed behind the diffuser 12. The infrared sensor 40 is merely a distance sensor which is used to measure a characteristic of the user's eye area. This infrared reflection measurement is very fast (from 1 to 100 khz) and allows the detection of high motion movements like a movement of the eye, a variation of the pupillary diameter or an eyelid blink.

As shown on figure 4, the infrared sensor 40 comprises a transmitter for transmitting a first signal 42 toward said at least one eye area 44 and a receptor for receiving a second signal 46 corresponding to the first signal 42 reflected by said at least one eye area 44. The controller 22 is configured to calculate how much infrared rays of the first signal 42 are reflected by the object in front of the infrared sensor 40. Different materials have different reflectivity so that it is possible to know that a different material is positioned in front of the infrared sensor 40 by comparing the difference between the first 42 and the second 46 signals. As an example, the reflectivity of the eye 48 and the reflectivity of the eyelids 50 are different. A variation between two consecutive second signals 46 thus occurs when the infrared rays are reflected first by the eye 48 and then by an eyelid 50. The same variation occurs when the infrared rays are reflected by different materials. It is thus possible to determine a variation of the position of one eyelid 50 or the pupil 52 as well as a variation of the size of the pupil 52. The variation of these characteristics may be representative of a visual discomfort of the user. It is therefore possible to determine a change in the visual comfort of the user depending on a variation of at least one of these characteristics.

Furthermore, the controller 22 may be configured to determine a variation of intensity and/or frequency of the second signal 42 and to correlate said variation to a response of the at least one eye area 44. Hence, it is possible to determine a variation of the characteristic either by comparing the intensity of the second signal 42 to a reference value and/or by detecting a short change in the second signal 42 value.

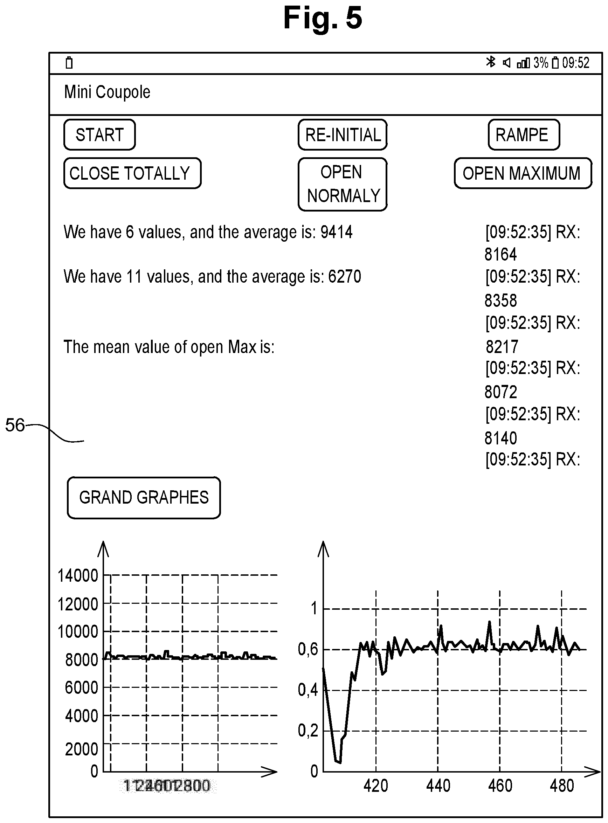

As shown on figure 5, the device 10 may be associated with an external terminal having a display for displaying information by means of an interface 56. The controller 22 is configured to communicate with said external terminal to transfer information representative of the acquired signal. In doing so, graphs 58 can be generated by the external terminal to show the evolution of the acquired signal.

Furthermore, the interface 56 may allow the user to command the calibration and measurement sequences to perform the determining method. This command can be made using a touch-sensitive screen or physical buttons of the external terminal. This external terminal may be a smartphone.

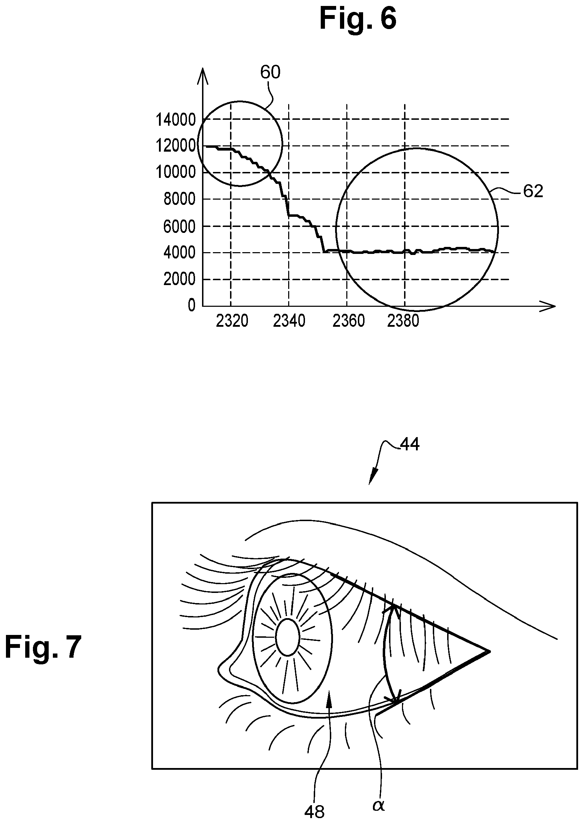

As an example, the graph 58 shown in figure 6 illustrates the variation of intensity of the signal acquired by the sensing unit 20. Said graph 58 shows the eye area 44 switching from an opening state 60 to a closing state 62.

The communication between the controller 22 and the external terminal is preferably wireless, e.g. using ©Bluetooth protocol or WIFI protocol. The controller 22 can thus communicate to the external terminal information related to the signal acquired and the external terminal can be used as a command to communicate instruction signals to the controller 22.

In this way, it is possible to visualize the values received from the infrared sensor 40 on the external terminal. Then, a calibration sequence may be instructed from the external terminal using the user interface 56. Values for different eye behaviors for different person may be obtained and recorded. A measurement sequence can be then performed to calculate other eye behaviors or detect specific eye behaviors using the recorded values.

As an alternative, the sensing unit 20 may comprise a plurality of infrared sensors 40 disposed either to face one eye area of the user or both eye areas of the user. In other words, the sensing unit 20 may be configured to acquire a plurality of signals representative of a plurality of characteristic of one eye area or at least one characteristic of both eye areas. Hence, the determination of a change in the visual comfort can be more complete and accurate. With two or more infrared sensors 40 facing the same eye area 44, it is thus possible to distinguish the direction of movement of the eyes and to discriminate these signals with those of an eyelid blink or a variation of pupillary diameter.

In the second embodiment, the sensing unit 20 comprises an image acquisition system configured to take bidimensional images of the eye area 44. The sensing unit 20 preferably takes images over time to perform the determining method over time. The image acquisition system may be a video camera.

In this embodiment, the controller 22 is configured to process the images of the eye area 44 acquired by the sensing unit 20 to determine the eye corner angle α, as shown on figure 7. The variation of the eye corner angle α can be correlated to a change in the visual comfort. Indeed, a quick variation of the position of the eyelids or the closing/opening state of the eye allows to define the eye behaviors. So, what we will do in this technology is to detect the eye corner angle of a person over time.

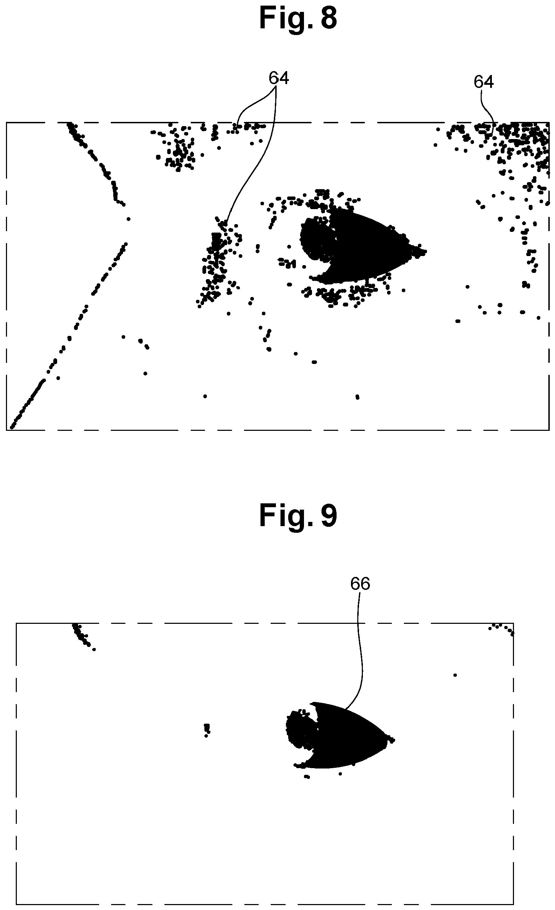

The controller 22 first transforms the image taken by the sensing unit 20 in RGB color model (Red Green Blue) into a YC_{b}Cᵣ color system. The image is then processed by applying intervals to find pixels which do not correspond to the skin of the user to obtain a processed image as shown on figure 8.

A dilatation is then applied to the image to withdraw pixels 64 forming noise to obtain an image as shown on figure 9 free from these pixels 64. The shape and the size of the target element is set to perform the dilation step. The image of figure 9 was obtained using an "ellipse" shape and a size of "4, 4" pixels (horizontal diameter and vertical diameter). The purpose is to isolate a group 66 of pixels corresponding to the eye 48 of the user.

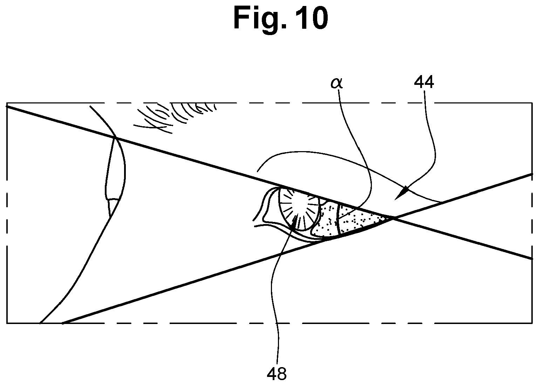

Then, last undesirable pixels are withdrawn by gathering the contacting pixels by groups and keeping the biggest group. This biggest group is considered to be the targeted area that would allow to determine the eye corner angle α. Contours of this biggest group are extracted and two lines following these contours are simulated, as shown on figure 10. The eye corner angle α is determined as the angle between these lines.

Then, all these processing steps are performed over time on each image taken by the sensing unit to make it possible to determine the eye corner angle α over time.

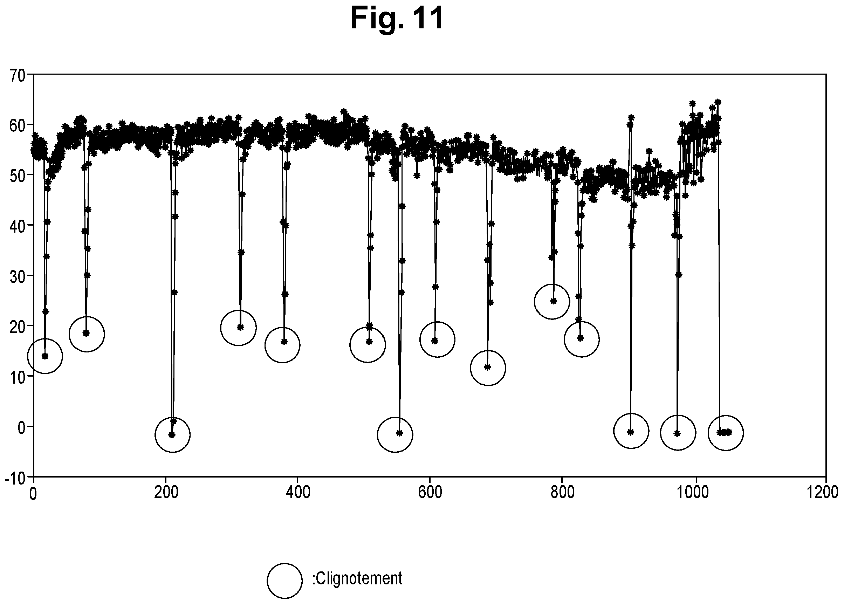

A calibration sequence is preferably performed before the measurement sequence to determine and record values of the eye corner angle α of the user as reference values. The controller 22 can then determine the closing/opening states of the eye or the position of the eyelids by comparing the eye corner angle α measured during the measurement sequence and the reference values. Figure 11 illustrates in a graph the evolution of the eye corner angle α determined by the controller 22. Every blink of the eye of the user corresponds to a low value of the eye corner angle α close to 0. It can also be seen that the eye angle corner α varies between 45° and 60° when the eye is in the opening state.

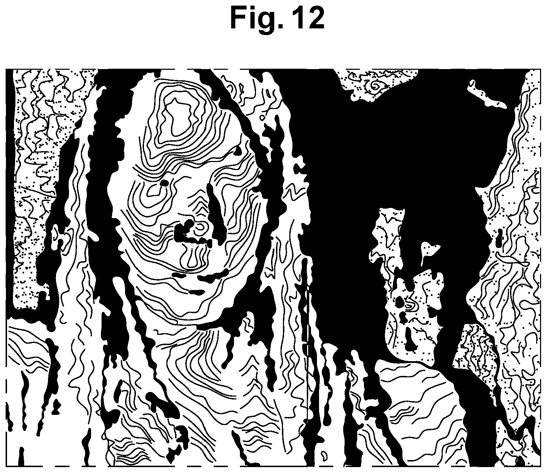

In the third embodiment, the sensing unit 20 comprises an image acquisition system configured to take tridimensional images of the eye area 44. The sensing unit 20 preferably takes images over time to perform the determining method over time. The controller 22 is configured to process the images of the eye area 44 acquired by the sensing unit 20 to determine the closing/opening state of the eyes of the user, as shown on figure 12. Said image acquisition system may be a video camera.

The method for determining a change in the visual comfort is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

For instance, the calibration sequence may be triggered upon user action, particularly using the switch 54 and the interface 56.

A typical example of computer-implementation of a method is to perform the method with a system adapted for this purpose. The system comprises a processor coupled to a memory. Optionally, the system may comprise a display for displaying a graphical user interface (GUI) as the interface 56, the memory having recorded thereon a computer program comprising instructions for performing the method. The memory may also store a database. The memory is any hardware adapted for such storage, possibly comprising several physical distinct parts (e.g. one for the program, and possibly one for the database).

By "database", it is meant any collection of data (i.e. information) organized for search and retrieval (e.g. a relational database, e.g. based on a predetermined structured language, e.g. SQL). When stored on a memory, the database allows a rapid search and retrieval by a computer. Databases are indeed structured to facilitate storage, retrieval, modification, and deletion of data in conjunction with various data-processing operations.

## Claims

1. A device (10) for determining a change in the visual comfort of a user, comprising:
• at least one light source (14) for stimulating at least one eye (48) of the user;
• a sensing unit (20) facing at least one eye area (44) of the user when the device (10) is worn by the user, the sensing unit (20) being configured to remotely acquire at least one signal (46) representative of at least one characteristic of said at least one eye area; and
• a controller (22) configured to determine a change in the visual comfort of the user depending on a variation of said at least one signal acquired by the sensing unit.

2. The device (10) according to claim 1, wherein said sensing unit is a contactless sensing unit (20) with regard to said at least one eye area for the acquisition of said at least one signal.

3. The device (10) according to claim 1 or 2, wherein said sensing unit comprises a transmitter for transmitting a first signal (42) toward said at least one eye area (44), and a receptor for receiving a second signal (46) corresponding to the first signal (42) reflected by said at least one eye area (44).

4. The device (10) according to any one of claims 1-3, wherein said controller (22) is configured to determine a variation of intensity and/or frequency of the second signal (46) and to correlate said variation to a response of the at least one eye area to a light stimulus provided by said at least light source.

5. The device (10) according to any one of the preceding claims, wherein said characteristic of said at least one eye area comprises at least one among a position of at least one eyelid (50), a position of the pupil (52) and a size of the pupil (52).

6. The device (10) according to any one of the preceding claims, wherein said controller (22) is configured to perform a calibration of the sensing unit (20) to obtain a first and a second values of the second signal (46) respectively corresponding to a first and a second states of the user's eye.

7. The device (10) according to claim 6, wherein the sensing unit further comprises a switch (54) reachable by the user to provide during said calibration to the controller (22) a signal representative of at least one characteristic of said at least one eye area when at least one user's eye area is in a first state and a signal representative of at least one characteristic of said at least one eye area from the switch when the user's area is in a second state.

8. The device (10) according to any one of the preceding claims, wherein said sensing unit (20) comprises at least one infrared sensor (40) facing said at least one eye area when the device (10) is worn by the user.

9. The device (10) according to any one of the preceding claims, wherein said sensing unit (20) comprises an image acquisition system for acquiring at least one image of said at least one eye area.

10. The device (10) according to claim 9, wherein said image acquisition system is configured to acquire at least one tridimensional image of said at least one eye area.

11. The device (10) according to any one of the preceding claims, wherein the device (10) is a binocular device, said at least one light source (14) being configured to stimulate at least one eye of the user.

12. The device (10) according to any one of the preceding claims, wherein said at least one eye area (44) comprises at least one among lower and upper eyelids (50), an eyebrow, an eyelash and an eye (48).

13. A method for determining a change in the visual comfort of the user, comprising the following steps:
- providing a device (10), comprising:
• at least one light source (14) for stimulating at least one eye (48) of the user;
• a sensing unit (20) facing at least one eye area (44) of the user when the device is worn by the user, the sensing unit being configured to remotely acquire at least one signal representative of at least one characteristic of said at least one eye area; and
• a controller (22) configured to determine a change in the visual comfort of the user depending on a variation of said at least one signal acquired by the sensing unit,
- positioning the device (10) on the user's head such that said sensing unit (20) faces at least one user's eye;
- emitting light toward at least one eye (48) of the user;
- remotely acquiring at least one measuring signal (46) representative of at least one characteristic of said at least one eye area;
- determining a change in the visual comfort of the user depending on a variation of said at least one measuring signal acquired (46).

14. The method according to claim 13, wherein the sensing unit (20) comprises a switch (54) reachable by the user, said method further comprising a calibration sequence comprising the following steps prior to the remotely acquiring step:
- receiving a signal representative of at least one characteristic of said at least one eye area from the switch when at least one user's eye area is in a first state,
- remotely acquiring a first calibration signal representative of said first state of said at least one eye area,
- determining a first value corresponding to the first state of the user's eye depending on the first calibration signal,
- receiving a signal representative of at least one characteristic of said at least one eye area from the switch when the user's area is in a second state,
- remotely acquiring a second calibration signal representative of said second state of said at least one eye area,
- determining a second value corresponding to the second state of the user's eye depending on the second calibration signal,
- recording the first and second values.

15. The method according to claim 13 or 14, wherein the method is a computer-implemented method.
